(19) Europäisches Patentamt
European Patent Office
Office européen des brevets

(11) Veröffentlichungsnummer: **0 115 607**
**A1**

(12) # EUROPÄISCHE PATENTANMELDUNG

(21) Anmeldenummer: **83112690.9**

(22) Anmeldetag: **16.12.83**

(51) Int. Cl.³: **C 07 D 401/06**, C 07 D 409/14, C 07 D 491/04, A 61 K 31/445 // (C07D491/04, 317/00, 209/00)

(30) Priorität: **04.01.83 DE 3300094**
**08.10.83 DE 3336643**

(43) Veröffentlichungstag der Anmeldung: **15.08.84**
**Patentblatt 84/33**

(84) Benannte Vertragsstaaten: **AT BE CH DE FR GB IT LI NL SE**

(71) Anmelder: **Merck Patent Gesellschaft mit beschränkter Haftung, Frankfurter Strasse 250, D-6100 Darmstadt (DE)**

(72) Erfinder: **Hausberg, Hans-Heinrich, Dr., Odenwaldstrasse 30, D-6105 Ober-Ramstadt (DE)**
Erfinder: **Böttcher, Henning, Dr., Soderstrasse 95, D-6100 Darmstadt (DE)**
Erfinder: **Gottschlich, Rudolf, Dr., Buchenweg 1, D-6107 Reinheim (DE)**
Erfinder: **Seyfried, Christoph, Dr., Mathildenstrasse 6, D-6104 Seeheim-Jugenheim (DE)**
Erfinder: **Minck, Klaus-Otto, Dr., Büchestrasse 8, D-6105 Ober-Ramstadt (DE)**

(54) **Tetrahydrocarbazolderivate.**

(57) Tetrahydrocarbazolderivate der allgemeinen Formel I

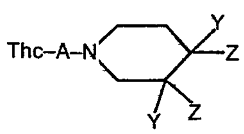

I

worin

Thc einen 1,2,3,4-Tetrahydrocarbazol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,

die beiden Reste Y jeweils H oder zusammen eine C–C-Bindung,

der eine Rest Z Ar,

der andere Rest Z H,

A $-CH_2-$ oder $-CH_2CH_2-$ und

Ar eine unsubstituierte oder ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituierte Phenylgruppe oder einen 2- oder 3-Thienylrest bedeuten und

worin die Alkylgruppen jeweils 1–4 C-Atome besitzen, sowie deren physiologisch unbedenkliche Säureadditionssalze zeigen Wirkungen auf das Zentralnervensystem.

EP 0 115 607 A1

Tetrahydrocarbazolderivate

Die Erfindung betrifft neue Tetrahydrocarbazolderivate der allgemeinen Formel I

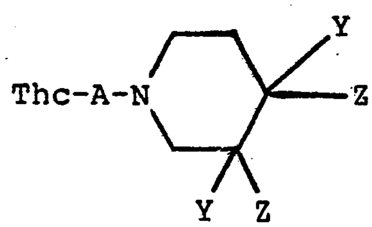

I

worin

Thc einen 1,2,3,4-Tetrahydrocarbazol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,

die beiden Reste Y jeweils H oder zusammen eine C-C-Bindung,

der eine Rest Z Ar,

der andere Rest Z H,

A $-CH_2-$ oder $-CH_2CH_2-$ und

Ar eine unsubstituierte oder ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituierte Phenylgruppe oder einen 2- oder 3-Thienylrest

bedeuten und

worin die Alkylgruppen jeweils 1 - 4 C-Atome besitzen,

sowie deren physiologisch unbedenkliche Säureadditionssalze.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem dopamin-stimulierende (Anti-Parkinson) Wirkungen. Im einzelnen induzieren die Verbindungen der Formel I contralaterales Drehverhalten in Hemiparkinson-Ratten (feststellbar nach der Methode von Ungerstedt et al., Brain Res. 24, (1970), 485-493) und hemmen die Bindung von tritiierten Dopaminagonisten und -antagonisten an striäre Rezeptoren (feststellbar nach der Methode von Schwarcz et al., J. Neurochemistry, 34, (1980), 772-778, und Creese et al., European J. Pharmacol., 46, (1977), 377-381). Zusätzlich hemmen die Verbindungen den Zungen-Kieferreflex bei der narkotisierten Ratte (feststellbar in Anlehnung an die Methoden von Barnett et al., European J. Pharmacol. 21, (1973), 178-182, und von Ilhan et al., European J. Pharmacol. 33, (1975) 61-64). Weiterhin treten analgetische und blutdrucksenkende Wirkungen auf; so wird nach intragastraler Gabe der Verbindungen der bei katheter-tragenden wachen spontan hypertonen Ratten (Stamm SHR/NIH-MO/CHB-EMD; Methode siehe Weeks und Jones, Proc. Soc. Exptl. Biol. Med. 104, (1960), 646-648) direkt gemessene arterielle Blutdruck gesenkt.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Tetrahydrocarbazolderivate der Formel I sowie ihre physiologisch unbedenklichen Säureadditionssalze.

In den Resten Thc und Ar bedeutet Alkyl vorzugsweise Methyl, ferner auch Ethyl, n-Propyl, Isopropyl, n-Butyl, Isobutyl, sek.-Butyl oder tert.-Butyl. O-Alkyl ist vorzugsweise Methoxy, ferner auch Ethoxy, n-Propoxy, Isopropoxy, n-Butoxy, Isobutoxy, sek.-Butoxy oder tert.-Butoxy. S-Alkyl steht vorzugsweise für Methylthio, aber auch für Ethylthio, n-Propylthio, Isopropylthio, n-Butylthio, Isobuthylthio, sek.-Buthylthio oder tert.-Butylthio. SO-Alkyl steht vorzugsweise für Methylsulfinyl, ferner auch für Ethylsulfinyl, n-Propylsulfinyl, Isopropylsulfinyl, n-Butylsulfinyl, Isobutylsulfinyl, sek.-Butylsulfinyl oder tert.-Butylsulfinyl. $SO_2$-Alkyl ist vorzugsweise Methylsulfonyl, ferner auch Ethylsulfonyl, n-Propylsulfonyl, Isopropylsulfonyl, n-Butylsulfonyl, Isobutylsulfonyl, sek.-Butylsulfonyl oder tert.-Butylsulfonyl.

Der Rest Thc bedeutet insbesondere einen unsubstituierten 1,2,3,4-Tetrahydrocarbazol-3-yl-rest. Falls Thc jedoch einen substituierten 1,2,3,4-Tetrahydrocarbazol-3-yl-rest bedeutet, so ist er vorzugsweise einfach, insbesondere in der 6- oder 7-Stellung substituiert. Weiterhin ist eine Substitution in 1-, 2-, 3-, 4-, 5-, 8- oder 9-Stellung möglich. Bevorzugte disubstituierte 1,2,3,4-Tetrahydrocarbazol-3-yl-reste sind in 6,7-Stellung substituiert; Disubstitution ist auch in 1,1-, 1,2-, 1,3-, 1,4-, 1,5-, 1,6-, 1,7-, 1,8-, 1,9-, 2,2-, 2,3-, 2,4-, 2,5-, 2,6-, 2,7-, 2,8-, 2,9-, 3,4-, 3,5-, 3,6-, 3,7-, 3,8-, 3,9-, 4,4-, 4,5-, 4,6-, 4,7-, 4,8-, 4,9-, 5,6-, 5,7-, 5,8-, 5,9-, 6,8-, 6,9-, 7,8-, 7,9- oder 8,9-Stellung möglich. In allen diesen Fällen können die Substituenten gleich oder verschieden sein.

Im einzelnen sind die bevorzugten Substituenten im Benzolring des Restes Thc Methyl, Ethyl, Methoxy, Ethoxy, Methylthio, Ethylthio, OH, F, Cl, Br, $CF_3$ und CN. Einige bevorzugte Bedeutungen des Restes Thc sind dementsprechend 1,2,3,4-Tetrahydrocarbazol-3-yl, ferner 1-, 2-, 3-, 4-, 5-, 6-, 7-, 8- oder 9-Methyl-1,2,3,4-tetrahydrocarbazol-3-yl, 1-, 2-, 3-, 4-, 5-, 6-, 7-, 8- oder 9-Ethyl-1,2,3,4-tetrahydrocarbazol-3-yl, 5-, 6-, 7- oder 8-Methoxy-1,2,3,4-tetrahydrocarbazol-3-yl, 5-, 6-, 7- oder 8-Ethoxy-1,2,3,4-tetrahydrocarbazol-3-yl, 5-, 6-, 7- oder 8-Methylthio-1,2,3,4-tetrahydrocarbazol-3-yl, 5-, 6-, 7- oder 8-Ethylthio-1,2,3,4-tetrahydrocarbazol-3-yl, 5-, 6-, 7- oder 8-Methylsulfinyl-1,2,3,4-tetrahydrocarbazol-3-yl, 5-, 6-, 7- oder 8-Methylsulfonyl-1,2,3,4-tetrahydrocarbazol-3-yl, 5-, 6-, 7- oder 8-Hydroxy-1,2,3,4-tetrahydrocarbazol-3-yl, 5-, 6-, 7- oder 8-Fluor-1,2,3,4-tetrahydrocarbazol-3-yl, 5-, 6-, 7- oder 8-Chlor-1,2,3,4-terahydrocarbazol-3-yl, 5-, 6-, 7- oder 8-Brom-1,2,3,4-tetrahydrocarbazol-3-yl, 5-, 6-, 7- oder 8-Trifluormethyl-1,2,3,4-tetrahydrocarbazol-3-yl, 5-, 6-, 7- oder 8-Cyan-1,2,3,4-tetrahydro-

carbazol-3-yl, 1,2-, 1,4-, 1,5-, 1,6-, 1,7-, 1,8-, 1,9-, 2,4-, 2,5-, 2,6-, 2,7-, 2,8-, 2,9-, 4,5-, 4,6-, 4,7-, 4,8-, 4,9-, 5,6-, 5,7-, 5,8-, 5,9-, 6,7-, 6,8-, 6,9-, 7,8-, 7,9- oder 8,9-Dimethyl-1,2,3,4-tetrahydrocarbazol-3-yl, 5-, 6-, 7- oder 8-Methoxy-9-methyl-1,2,3,4-tetrahydrocarbazol-3-yl, 5-, 6-, 7- oder 8-Methylthio-9-methyl-1,2,3,4-tetrahydrocarbazol-3-yl, 5-, 6-, 7- oder 8-Fluor-9-methyl-1,2,3,4-tetrahydrocarbazol-3-yl, 5-, 6-, 7- oder 8-Chlor-9-methyl-1,2,3,4-tetrahydrocarbazol-3-yl, 5-, 6-, 7- oder 8-Brom-9-methyl-1,2,3,4-tetrahydrocarbazol-3-yl, 5-, 6-, 7- oder 8-Trifluormethyl-9-methyl-1,2,3,4-tetrahydrocarbazol-3-yl, 5-, 6-, 7- oder 8-Cyan-9-methyl-1,2,3,4-tetrahydrocarbazol-3-yl, 1- oder 2-Methyl-5-, -6-, -7- oder -8-methoxy-1,2,3,4-tetrahydrocarbazol-3-yl, 1- oder 2-Methyl-5-, -6-, -7- oder -8-methylthio-1,2,3,4-tetrahydrocarbazol-3-yl, 1- oder 2-Methyl-5-, -6-, -7- oder -8-fluor-1,2,3,4-tetrahydrocarbazol-3-yl, 1- oder 2-Methyl-5-, -6-, -7- oder -8-chlor-1,2,3,4-tetrahydrocarbazol-3-yl, 1- oder 2-Methyl-5-, -6-, -7- oder -8-brom-1,2,3,4-tetrahydrocarbazol-3-yl, 1- oder 2-Methyl-5-, -6-, -7- oder -8-trifluormethyl-1,2,3,4-tetrahydrocarbazol-3-yl, 1- oder 2-Methyl-5-, -6-, -7- oder -8-cyan-1,2,3,4-tetrahydrocarbazol-3-yl, 6-Methyl-7-fluor-1,2,3,4-tetrahydrocarbazol-3-yl, 6-Fluor-7- oder -9-methyl-1,2,3,4-tetrahydrocarbazol-3-yl, 6-Methyl-7-chlor-1,2,3,4-tetrahydrocarbazol-3-yl, 6-Chlor-7-methyl-1,2,3,4-tetrahydrocarbazol-3-yl, 5-Chlor-6- oder -7-methyl-1,2,3,4-tetrahydrocarbazol-3-yl, 5,6-, 5,7-, 5,8-, 6,7-, 6,8- oder 7,8-Dimethoxy-1,2,3,4-tetrahydrocarbazol-3-yl, 5,6-, 5,7-, 5,8-, 6,7-, 6,8- oder 7,8-Dichlor-1,2,3,4-tetrahydrocarbazol-3-yl, 5-Trifluormethyl-6-, -7- oder-8-chlor-1,2,3,4-tetrahydrocarbazol-3-yl, 5,6-, 6,7- oder 7,8-Methylendioxy-1,2,3,4-tetrahydrocarbazol-3-yl.

Der Rest A ist vorzugsweise $-CH_2-$.

Der Rest Ar ist bevorzugt unsubstituiertes Phenyl. Falls Ar eine substituierte Phenylgruppe bedeutet, so ist diese vorzugsweise einfach substituiert. Sie kann jedoch auch zweifach substituiert sein, wobei die Substituenten gleich oder verschieden sein können. Bevorzugte Substituenten an der Phenylgruppe sind Methyl, F, Cl, Br und Trifluormethyl. Im einzelnen ist Ar bevorzugt Phenyl, o-, m- oder p-Fluorphenyl, o-, m- oder p-Chlorphenyl, o-, m- oder p-Bromphenyl, o-, m- oder p-Tolyl, o-, m- oder p- Methoxyphenyl, o-, m- oder p-Trifluormethylphenyl, ferner z. B. o-, m- oder p-Ethylphenyl, o-, m- oder p-n-Propylphenyl, o-, m- oder p-Isopropylphenyl, o-, m- oder p-n-Butylphenyl, o-, m- oder p-Isobutylphenyl, o-, m- oder p-Jodphenyl, weiterhin Dihalogenphenyl wie 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Fluor-4-chlorphenyl, 2-Brom-4-chlorphenyl; Dimethylphenyl wie 2,3-, 2,4-, 2,5-, 2,6-, 3,4- oder 3,5-Dimethylphenyl; Methyl-chlorphenyl wie 2-Methyl-4-chlorphenyl; Dimethoxyphenyl wie 2,3-, 2,4-, 2,5-, 2,6-, 3,4- oder 3,5-Dimethoxyphenyl; 2,3- oder 3,4-Methylendioxyphenyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ig ausgedrückt werden, die der Formel I entsprechen und worin die nicht näher bezeichneten Reste die bei Formel I angegebene

Bedeutung haben, worin jedoch

in Ia Thc 1,2,3,4-Tetrahydrocarbazol-3-yl, Methyl-1,2,3,4-tetrahydrocarbazol-3-yl, Methoxy-1,2,3,4-tetrahydrocarbazol-3-yl, Dimethoxy-1,2,3,4-tetrahydrocarbazol-3-yl, Hydroxy-1,2,3,4-tetrahydrocarbazol-3-yl, Dihydroxy-1,2,3,4-tetrahydrocarbazol-3-yl, Fluor-1,2,3,4-tetrahydrocarbazol-3-yl, Chlor-1,2,3,4-tetrahydrocarbazol-3-yl, Dichlor-1,2,3,4-tetrahydrocarbazol-3-yl, Brom-1,2,3,4-tetrahydrocarbazol-3-yl,Cyan-1,2,3,4-tetrahydrocarbazol-3-yl oder Methylendioxy-1,2,3,4-tetrahydrocarbazol-3-yl bedeutet, wobei die Substituenten vorzugsweise in 6- und/oder 7-Stellung stehen;

in Ib Thc 1,2,3,4-Tetrahydrocarbazol-3-yl, 6- oder 7-Methyl-1,2,3,4-tetrahydrocarbazol-3-yl, 6,7-Dimethyl-1,2,3,4-tetrahydrocarbazol-3-yl, 6- oder 7-Methoxy-1,2,3,4-tetrahydrocarbazol-3-yl, 6,7-Dimethoxy-1,2,3,4-tetrahydrocarbazol-3-yl, 6- oder 7-Chlor-1,2,3,4-tetrahydrocarbazol-3-yl, 6- oder 7-Hydroxy-1,2,3,4-tetrahydrocarbazol-3-yl, 6,7- Dihydroxy-1,2,3,4-tetrahydrocarbazol-3-yl, oder 6,7-Methylendioxy-1,2,3,4-tetrahydro-carbazol-3-yl bedeutet;

in Ic A $-CH_2-$ bedeutet;

in Id Ar Phenyl, Tolyl, Methoxyphenyl, Fluorphenyl, Chlorphenyl, Trifluormethylphenyl oder Chlortrifluormethylphenyl bedeutet;

in Ie Ar Phenyl bedeutet

in If Thc 1,2,3,4-Tetrahydrocarbazol-3-yl, 6- oder 7-Methoxy-1,2,3,4-tetrahydrocarbazol-3-yl, 6- oder 7-Hydroxy-1,2,3,4-tetrahydrocarbazol-3-yl, 6,7-Dimethoxy-1,2,3,4-tetrahydrocarbazol-3-yl, 6- oder 7-Chlor-1,2,3,4-tetrahydrocarbazol-3-yl oder 6,7-Dihydroxy-1,2,3,4-tetrahydrocarbazol-3-yl und

Ar Phenyl

bedeuten;

in Ig Thc 1,2,3,4-Tetrahydrocarbazol-3-yl, 6- oder 7-Hydroxy-1,2,3,4-tetrahydrocarbazol-3-yl

A $-CH_2-$ und

Ar Phenyl

bedeuten.

Die Verbindungen der Formel I besitzen ein asymmetrisches Kohlenstoffatom in 3-Stellung des Tetrahydrocarbazolrings. Sie können weitere asymmetrische Kohlenstoffatome besitzen. Sie können daher als Racemate, falls mehrere asymmetrische Kohlenstoffatome vorhanden sind, auch als Gemische mehrere Racemate sowie in verschiedenen optisch-aktiven Formen vorliegen.

Gegenstand der Erfindung ist ferner ein Verfahren zur Herstellung der Verbindungen der Formel I sowie ihrer physiologisch unbedenklichen Säureadditionssalze, dadurch gekennzeichnet, daß man eine Verbindung der allgemeinen Formel II

$Thc\text{-}A\text{-}X^1$ II

worin

$X^1$ X oder $NH_2$ und

X Cl, Br, J, OH oder eine reaktionsfähig funktionell abgewandelte OH-Gruppe bedeuten und

Thc und A die angegebenen Bedeutungen haben,

mit einer Verbindung der allgemeinen Formel III

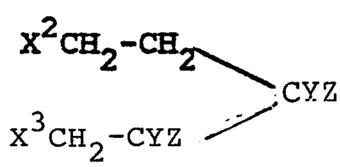

III

worin

$X^2$ und $X^3$ gleich oder verschieden sein können und, falls $X^1 = NH_2$ ist, jeweils X, andernfalls zusammen NH bedeuten und

Y und Z die angegebene Bedeutung haben

umsetzt

oder daß man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt oder daß man eine sonst der Formel I entsprechende Verbindung, die jedoch an Stelle eines oder mehrerer Wasserstoffatome eine oder mehrere solvolytisch abspaltbare Gruppe(n) enthält,

mit einem solvolysierenden Mittel behandelt oder daß man zur Herstellung von Verbindungen der Formel I, worin die beiden Reste Y zusammen eine C-C-Bindung bedeuten, eine Verbindung der Formel IV

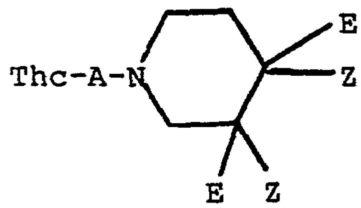

IV

worin

| | |
|---|---|
| der eine Rest E | X, CN oder $NH_2$, |
| der andere Rest E | H bedeutet und |
| Thc, A, X und Z | die angegebenen Bedeutungen haben |

mit einem HE-abspaltenden Mittel behandelt und/oder daß man gegebenenfalls in einer Verbindung der Formel I eine Thioethergruppe zu einer SO-Gruppe oder $SO_2$-Gruppe oder eine SO-Gruppe zu einer $SO_2$-Gruppe oxydiert und/oder eine Alkoxygruppe unter Bildung einer OH-Gruppe spaltet und/oder eine C-C-Doppelbindung hydriert und/oder daß man eine erhaltene Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

In den Tetrahydrocarbazolderivaten der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1 - 6 (z. B. Methansulfonyloxy) oder Arylsulfonyloxy mit 6 - 10 C-Atomen (z. B. Benzolsulfonyloxy, p-Toluolsulfonyloxy, 1- oder 2-Naphthalin-sulfonyloxy).

Dementsprechend sind die Tetrahydrocarbazolderivate der Formel I insbesondere durch Umsetzung der Verbindungen der Formeln Thc-A-Cl oder Thc-A-Br mit Piperidinderivaten der Formel III, worin $X^2$ und $X^3$ zusammen eine NH-Gruppe bedeuten (nachstehend als IIIa bezeichnet) erhältlich.

Die Verbindungen der Formeln II und insbesondere III sind zum Teil bekannt; die nicht bekannten Verbindungen der Formeln II und III können leicht analog zu den bekannten Verbindungen hergestellt werden.

So sind die Verbindungen der Formel II (A = $-CH_2-$) beispielsweise erhältlich, indem man die meist bekannten, aus den entsprechenden Phenylhydrazinen und den entsprechenden Cyclohexanon-4-carbonsäureestern mit Hilfe der Fischer-Indolsynthese herstellbaren Carbonsäuren der Formel Thc-COOH zu den entsprechenden Carbinolen der Formel $Thc-CH_2OH$ reduziert und diese, z. B. mit $SOCl_2$, zu den entsprechenden Chloriden der Formel $Thc-CH_2Cl$ bzw., z. B. mit $PBr_3$, zu den entsprechenden Bromiden der Formel $Thc-CH_2Br$

umsetzt. Reaktion der letztgenannten Chloride oder Bromide mit KCN führt zu den Acetonitrilen der Formel Thc-$CH_2CN$, die zu den Essigsäuren der Formel Thc-$CH_2COOH$ hydrolysiert werden können. Reduktion und weitere analoge Umsetzungen gibt Verbindungen der Formeln Thc-$CH_2CH_2OH$, Thc-$CH_2CH_2Cl$ und Thc-$CH_2CH_2Br$.

Die Jodverbindungen der Formel Thc-A-J, z. B. 3-Jodmethyl-1,2,3,4-tetrahydrocarbazol, sind z. B. durch Einwirkung von Kaliumjodid auf die zugehörigen p-Toluolsulfonsäureester erhältlich. Die Amine der Formel Thc-A-$NH_2$ sind z. B. aus den Halogeniden mit Phthalimidkalium oder durch Reduktion der entsprechenden Nitrile zugänglich.

Die Piperidinderivate IIIa sind größtenteils bekannt (vgl. DE-OS 20 60 816) und z.B. erhältlich durch Umsetzung von 3- oder 4-Piperidon mit metallorganischen Verbindungen der Formel M-Ar (worin M ein Li-Atom oder MgHal bedeutet), anschließende Hydrolyse zu den entsprechenden 3-Ar-3- bzw. 4-Ar-4-hydroxy-piperidinen sowie gewünschtenfalls nachfolgende Dehydratisierung zu 3-Ar- bzw. 4-Ar-3,4-dehydro-piperidinen sowie gewünschtenfalls Hydrierung zu 3-Ar- bzw. 4-Ar-piperidinen. Verbindungen der Formel III ($X^2$ und $X^3$ = jeweils X) sind z.B. herstellbar durch Reduktion von 2- oder 3-Ar-glutarsäureestern bzw. 2- oder 3-Ar-2-penten-1,5-disäurediestern zu 2- oder 3-Ar-1,5-pentandiolen bzw. zu 2- oder 3-Ar-2-penten-1,5-diolen und gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z. B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Thc-A-$NH_2$ bzw. des Piperidinderivates der Formel IIIa kann günstig sein. Die Reaktionszeit liegt je nach den angewendeten Bedingungen zwischen einigen Minuten und 14 Tagen, die Reaktionstemperatur zwischen etwa 0 und 150 $^{o}$, normalerweise zwischen 20 und 130 $^{o}$.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit reduzierenden Mitteln behandelt, vorzugsweise bei Temperaturen zwischen -80 und +250 $^{o}$ in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z. B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel V

Thc'-L-Q-Ar' V

worin

Thc' einen 1,2,3,4-Tetrahydrocarbazol-3-yl-rest, der ein- oder zweifach durch Alkyl, O-Alkyl, S-Alkyl, SO-Alkyl, $SO_2$-Alkyl, OH, F, Cl, Br, $CF_3$, CN und/oder O-Benzyl oder durch eine Methylendioxygruppe und/oder durch eine Arylsulfonylgruppe oder eine Benzylgruppe in 9-Stellung substituiert sein kann,

L $-CH_2-$, $-CH_2CH_2-$, $-CO-$, $-CH_2CO-$, $-COCH_2-$ oder $-COCO-$,

Q 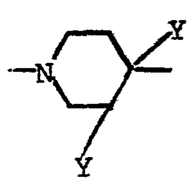 , 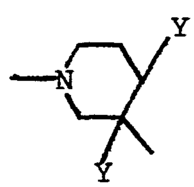 , 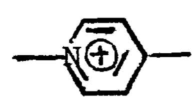 $An^{\ominus}$ oder

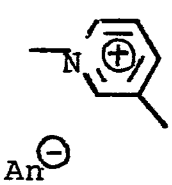 $An^{\ominus}$

$An^{\ominus}$ ein Anion einer starken Säure und

Ar' eine unsubstituierte oder ein- oder zweifach durch Alkyl, O-Alkyl, S-Alkyl, SO-Alkyl, $SO_2$-Alkyl, OH, F, Cl, Br, $CF_3$, CN und/oder O-Benzyl oder durch eine Methylendioxygruppe

substituierte Phenylgruppe oder einen 2- oder 3-Thienylrest bedeuten,

worin jedoch nicht gleichzeitig Thc' = Thc, L = A,

Q = 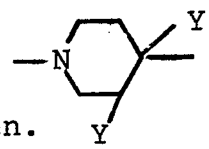 oder 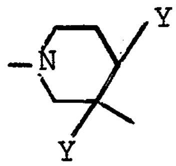 und Ar' = Ar sein können.

In den Verbindungen der Formel V ist L bevorzugt -CO- oder $-CH_2CO-$.

Verbindungen der Formel V sind z.B. herstellbar durch Umsetzung eines 3- oder 4-Ar'-piperidins, -1,2,3,6-tetrahydropyridins oder -pyridins mit einer Verbindung der Formel VI

$$Thc'-L-X^1 \qquad VI$$

worin

Ar', Thc', L und $X^1$ die oben angegebenen Bedeutungen haben,

unter den Bedingungen, die oben für die Umsetzung von II mit III angegeben sind. Die Herstellung von Säureamiden der Formeln V (L = -CO- oder $-CH_2CO-$,

Q = 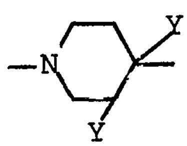 oder 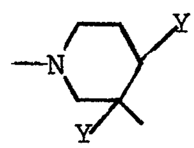)

gelingt z.B. aus den freien Carbonsäuren der Formeln Thc-COOH oder $Thc-CH_2-COOH$ und Piperidinen der Formel IIIa in Gegenwart eines Dehydratisierungsmittels, z.B. Carbonyldiimidazol oder Dicyclohexylcarbodiimid in einem der angegebenen inerten Lösungsmittel, bevorzugt THF.

Wird als Reduktionsmittel nascierender Wasserstoff verwendet, so kann man diesen z. B. durch Behandlung von Metallen mit schwachen Säuren oder mit Basen erzeugen. So kann man z. B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerigalkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether with Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150 °, insbesondere zwischen etwa 0 und etwa 100 °.

Besonders vorteilhaft lassen sich -CO-Gruppen in Säureamiden mit $LiAlH_4$ in THF bei Temperaturen zwischen etwa 0 und 66° zu $CH_2$-Gruppen reduzieren. Dabei können in 9-Stellung des 1,2,3,4-Tetrahydrocarbazolrings befindliche Arylsulfonyl-Schutzgruppen gleichzeitig reduktiv abgespalten werden.

Eine Reduktion der Pyridiniumsalze der Formel V

(worin Q 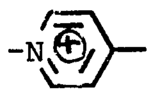 $An^{\ominus}$ oder 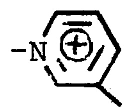 $An^{\ominus}$

und An vorzugsweise Cl oder Br bedeutet) zu Verbindungen der Formel I gelingt z.B. mit $NaBH_4$ in Wasser, Methanol, Ethanol oder in Gemischen dieser Lösungsmittel, falls erwünscht unter Zusatz einer Base wie NaOH, bei Temperaturen zwischen etwa 0 und 80°.

N-Benzylgruppen können reduktiv mit Natrium in flüssigem Ammoniak abgespalten werden.

Es ist ferner möglich, eine oder mehrere Carbonylgruppen nach der Methode von Wolff-Kishner zu $CH_2$-Gruppen zu reduzieren, z. B. durch Behandlung mit wasserfreiem Hydrazin in absolutem Ethanol unter Druck bei Temperaturen zwischen etwa 150 und 250 °. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3 - 4 Stunden gekocht. Anschließend wird das Wasser abdestilliert und das gebildete Hydrazon

bei Temperaturen bis zu etwa 200 ° zersetzt. Die Wolff-Kishner-Reduktion kann auch bei Raumtemperatur in Dimethylsulfoxid mit Hydrazin ausgeführt werden.

Verbindungen, die sonst der Formel I entsprechen, aber an Stelle eines oder mehrerer H-Atome eine oder mehrere solvolytisch abspaltbare Gruppe(n) enthalten, können zu den Verbindungen der Formel I solvolysiert, insbesondere hydrolysiert werden. Die Ausgangsstoffe für die Solvolyse sind beispielsweise erhältlich durch Reaktion von IIIa mit Verbindungen, die der Formel II ($X^1 = X$) entsprechen, aber an Stelle eines oder mehrerer H-Atome eine oder mehrere solvolytisch abspaltbare Gruppe(n) enthalten. So können 1-Acyl-1,2,3,4-tetrahydrocarbazolderivate (entsprechend der Formel I, aber in 9-Stellung des Thc-Restes eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des 1,2,3,4-Tetrahydrocarbazolringes unsubstituierten 1,2,3,4-Tetrahydrocarbazolderivaten hydrolysiert werden, z. B. in saurem, besser in neutralem oder alkalischem Medium bei Temperaturen zwischen 0 und 200 °. Als basische Katalysatoren verwendet man zweckmäßig Natrium-, Kalium- oder Calciumhydroxid, Natrium- oder Kaliumcarbonat, oder Ammoniak. Als Lösungsmittel wählt man vorzugsweise Wasser, niedere Alkohole wie Methanol, Ethanol; Ether wie THF, Dioxan; Sulfone wie Tetramethylensulfon; oder deren Gemische. Eine Hydrolyse kann auch bereits beim Behandeln mit Wasser allein erfolgen, insbesondere in der Siedehitze.

Man gelangt ferner zu Verbindungen der Formel I, worin die beiden Reste Y zusammen eine C-C-Bindung bedeuten, indem man aus Verbindungen der Formel IV unter Ausbildung einer Doppelbindung HE abspaltet. Entsprechend der Definition von E kann es sich z.B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel IV sind z.B. erhältlich durch Umsetzung von II ($X^1 = X$) mit einer Verbindung der Formel IX

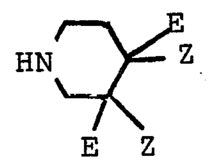

IX

worin E und Z die angegebenen Bedeutungen haben.

Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z. B. Kalium-tert.-butylat, Amine, wie z. B. Dimethylanilin, Pyridin, Collidin oder Chinolin; als Lösungmittel benutzt man z. B. Benzol, Toluol, Cyclohexan, Methanol, Dioxan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z. B. Wasser oder Ethanol) kann von Vorteil sein. Die Eliminierung von Acyl-, Alkylsulfonyl-

sowie Alkoxysulfonyloxy- oder Aminoresten kann unter ähnlichen Bedingungen durchgeführt werden. Eine Eliminierung von Sulfonsäure-resten, z. B. die der Mesylate oder Tosylate, erfolgt schonend durch Kochen in DMF oder Dimethylsulfoxid mit Alkalimetallcarbonaten z. B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel IV (eine Gruppe E = CN) durch Erhitzen abgespalten werden. Die Eliminierung von HE aus IV erfolgt allgemein bei Temperaturen zwischen etwa 0 und etwa 250 °, vorzugsweise zwischen 50 und 200 °.

Weiterhin kann man in einem Thioether der Formel I die Thioethergruppe zu einer SO-Gruppe oder zu einer $SO_2$-Gruppe oder in einem Sulfoxid der Formel I die SO-Gruppe zu einer $SO_2$-Gruppe oxydieren. Die zu oxydierenden Thioether- oder Sulfoxidgruppen können als Substituenten im Rest Thc und/oder im Rest Ar vorhanden sein. Will man die Sulfoxide erhalten, so oxydiert man beispielsweise mit Wasserstoffperoxid, Persäuren wie m-Chlorperbenzoesäure, Cr(VI)-Verbindungen wie Chromsäure, $KMnO_4$, 1-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismäßig milden Bedingungen und bei relativ niedrigen Temperaturen (etwa -80 bis +100 °). Will man dagegen die Sulfone (aus den Thioethern oder den Sulfoxiden) erhalten, so verwendet man die gleichen Oxydationsmittel unter kräftigeren Bedingungen und/oder im

Überschuß sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohol wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$. Ein bevorzugtes Oxydationsmittel ist 30%iges wässeriges Wasserstoffperoxid. Dieses führt bei Anwendung der berechneten Menge in Lösungsmitteln wie Essigsäure, Aceton, Methanol, Ethanol oder wässeriger Natronlauge bei Temperaturen zwischen -20 und 100 $^{o}$ zu den Sulfoxiden, im Überschuß bei höheren Temperaturen, vorzugsweise in Essigsäure oder in einem Gemisch aus Essigsäure und Acetanhydrid, zu den Sulfonen.

Ether der Formel I, in denen die Reste Thc und/oder Ar ein- oder zweifach durch O-Alkyl substituiert sind, können nach Methoden, die aus der Literatur bekannt sind, gespalten werden, wobei die entsprechenden Hydroxyderivate entstehen. Z. B. kann man die Ether spalten durch Behandeln mit HBr oder HJ in wässeriger oder essigsaurer Lösung, durch Erhitzen mit Lewis-Säuren wie $AlCl_3$ oder Bortrihalogeniden oder durch Verschmelzen mit Pyridin- oder Anilin-hydrohalogeniden, vorzugsweise Pyridinhydrochlorid, bei etwa 150 - 250 $^{o}$. Besonders schonend ist die reduktive Spaltung mit Diisobutylaluminiumhydrid (Methode vgl. Synthesis 1975, 617).

Falls erwünscht, können ungesättigte Verbindungen der Formel I, worin die beiden Reste Y zusammen eine C-C-Bindung bedeuten, zu den entsprechenden gesättigten Verbindungen der Formel I, worin die beiden Reste Y jeweils H bedeuten, hydriert werden, zweckmäßig in Gegenwart eines Schwermetallkatalysators wie Platin, Palladium oder Raney-Nickel in einem inerten Lösungsmittel, z.B. einem Alkohol wie Methanol oder Ethanol bei Temperaturen zwischen etwa 0 und 150° und Drucken zwischen etwa 1 und 200 bar.

Eine erhaltene Base der Formel I kann mit einer Säure in das zugehörige Säureadditionssalz übergeführt werden. Für diese Umsetzung eignen sich Säuren die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen

 

Lyophilisate z. B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Gegenstand der Erfindung ist ferner die Verwendung der Verbindungen der Formel I und ihrer physiologisch unbedenklichen Salze bei der therapeutischen Behandlung des menschlichen oder tierischen Körpers und bei der Bekämpfung von Krankheiten, insbesondere von Parkinsonismus, von extrapyramidalen Störungen bei der Neuroleptikatherapie, von Depressionen und/oder Psychosen und von Nebenwirkungen bei der Behandlung der Hypertonie (z. B. mit $\alpha$-Methyldopa). Ferner können die Verbindungen in der Endokrinologie und Gynäkologie Verwendung finden, z. B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z. B. Migräne), insbesondere in der Geriatrie ähnlich wie gewisse Ergot-Alkaloide.

Dabei werden die erfindungsgemäßen Substanzen in der Regel in Analogie zu bekannten, im Handel befindlichen Präparaten (z. B. Bromocriptin; Dihydroergocornin) verabreicht, vorzugsweise in Dosierungen zwischen etwa 0,2 und 500 mg, insbesondere zwischen 0,2 und

50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Bevorzugte Dosierungsbereiche für einzelne Indikationen sind folgende: Parkinsonismus 1 bis 200, vorzugsweise 40 bis 100; Dyskinesie 40 bis 100; Psychose, z.B. chronische Schizophrenie 2 bis 20; Akromegalie 2 bis 50 mg pro Dosierungseinheit. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und-weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Toluol, Chloroform oder Dichlormethan, trennt ab, trocknet die organische Phase über Natriumsulfat, filtriert, dampft ein und reinigt durch Chromatographie und/oder Kristallisation. Temperaturen sind in Celsiusgraden angegeben. Rf-Werte an Kieselgel ($CH_2Cl_2$/Methanol 95 : 5, wenn nicht anders angegeben).

Beispiel 1

Man rührt eine Lösung von 2,19 g 3-Chlormethyl-1,2,3,4-tetrahydrocarbazol (oder 2,63 g 3-Brommethyl-1,2,3,4-tetrahydrocarbazol (erhältlich durch Reduktion von 1,2,3,4-Tetrahydrocarbazol-3-carbonsäure mit $LiAlH_4$ zu 3-Hydroxymethyl-1,2,3,4-tetrahydrocarbazol und nachfolgende Umsetzung mit $SOCl_2$ oder $PBr_3$) und 1,6 g 4-Phenyl-1,2,3,6-tetrahydropyridin in 10 ml Acetonitril 12 Stunden bei 20 °, arbeitet wie üblich auf und erhält 3-(4-Phenyl-1,2,3,6-tetrahydropyridyl-1-methyl)-1,2,3,4-tetrahydrocarbazol ("P"). F. 163-165 °.

Analog erhält man aus den entsprechenden 3-Chloralkyl- bzw. 3-Bromalkyl-1,2,3,4-tetrahydrocarbazolen, z. B.

3-Chlormethyl-6-methoxy-1,2,3,4-tetrahydrocarbazol
3-Chlormethyl-7-methoxy-1,2,3,4-tetrahydrocarbazol
3-Chlormethyl-6-hydroxy-1,2,3,4-tetrahydrocarbazol
3-Chlormethyl-7-hydroxy-1,2,3,4-tetrahydrocarbazol
3-Chlormethyl-6,7-dimethoxy-1,2,3,4-tetrahydrocarbazol
3-(2-Chlorethyl)-1,2,3,4-tetrahydrocarbazol
3-(2-Chlorethyl)-6-methoxy-1,2,3,4-tetrahydrocarbazol
3-(2-Chlorethyl)-7-methoxy-1,2,3,4-tetrahydrocarbazol
3-(2-Chlorethyl)-6-hydroxy-1,2,3,4-tetrahydrocarbazol
3-(2-Chlorethyl)-7-hydroxy-1,2,3,4-tetrahydrocarbazol
3-(2-Chlorethyl)-7-chlor-1,2,3,4-tetrahydrocarbazol
3-(2-Chlorethyl)-6,7-dimethoxy-1,2,3,4-tetrahydrocarbazol

mit den entsprechenden 4-Aryl-1,2,3,6-tetrahydropyridinen:

3-(4-Phenyl-1,2,3,6-tetrahydropyridyl-1-methyl)-6-methyl-1,2,3,4-tetrahydrocarbazol
3-(4-Phenyl-1,2,3,6-tetrahydropyridyl-1-methyl)-7-methyl-1,2,3,4-tetrahydrocarbazol
3-(4-Phenyl-1,2,3,6-tetrahydropyridyl-1-methyl)-9-methyl-1,2,3,4-tetrahydrocarbazol

3-(4-Phenyl-1,2,3,6-tetrahydropyridyl-1-methyl)-6-methoxy-1,2,3,4-tetrahydrocarbazol, F. 161-163°

3-(4-Phenyl-1,2,3,6-tetrahydropyridyl-1-methyl)-7-methoxy-1,2,3,4-tetrahydrocarbazol, F. 164 - 166°

3-(4-Phenyl-1,2,3,6-tetrahydropyridyl-1-methyl)-8-methoxy-1,2,3,4-tetrahydrocarbazol

3-(4-Phenyl-1,2,3,6-tetrahydropyridyl-1-methyl)-6-ethoxy-1,2,3,4-tetrahydrocarbazol

3-(4-Phenyl-1,2,3,6-tetrahydropyridyl-1-methyl)-6-methylthio-1,2,3,4-tetrahydrocarbazol

3-(4-Phenyl-1,2,3,6-tetrahydropyridyl-1-methyl)-7-methylthio-1,2,3,4-tetrahydrocarbazol

3-(4-Phenyl-1,2,3,6-tetrahydropyridyl-1-methyl)-6-methylsulfinyl-1,2,3,4-tetrahydrocarbazol

3-(4-Phenyl-1,2,3,6-tetrahydropyridyl-1-methyl)-7-methylsulfinyl-1,2,3,4-tetrahydrocarbazol

3-(4-Phenyl-1,2,3,6-tetrahydropyridyl-1-methyl)-6-methylsulfonyl-1,2,3,4-tetrahydrocarbazol

3-(4-Phenyl-1,2,3,6-tetrahydropyridyl-1-methyl)-7-methylsulfonyl-1,2,3,4-tetrahydrocarbazol

3-(4-Phenyl-1,2,3,6-tetrahydropyridyl-1-methyl)-5-hydroxy-1,2,3,4-tetrahydrocarbazol

3-(4-Phenyl-1,2,3,6-tetrahydropyridyl-1-methyl)-6-hydroxy-1,2,3,4-tetrahydrocarbazol, Rf 0,2 (Toluol/Triethylamin 9:1)

3-(4-Phenyl-1,2,3,6-tetrahydropyridyl-1-methyl)-7-hydroxy-1,2,3,4-tetrahydrocarbazol, Rf 0,28 ($CH_2Cl_2$/Methanol 9:1)

3-(4-Phenyl-1,2,3,6-tetrahydropyridyl-1-methyl)-8-hydroxy-1,2,3,4-tetrahydrocarbazol

3-(4-Phenyl-1,2,3,6-tetrahydropyridyl-1-methyl)-6-fluor-1,2,3,4-tetrahydrocarbazol

3-(4-Phenyl-1,2,3,6-tetrahydropyridyl-1-methyl)-7-fluor-1,2,3,4-tetrahydrocarbazol

3-(4-Phenyl-1,2,3,6-tetrahydropyridyl-1-methyl)-6-chlor-1,2,3,4-tetrahydrocarbazol

3-(4-Phenyl-1,2,3,6-tetrahydropyridyl-1-methyl)-7-chlor-1,2,3,4-tetrahydrocarbazol
3-(4-Phenyl-1,2,3,6-tetrahydropyridyl-1-methyl)-6-brom-1,2,3,4-tetrahydrocarbazol
3-(4-Phenyl-1,2,3,6-tetrahydropyridyl-1-methyl)-7-brom-1,2,3,4-tetrahydrocarbazol
3-(4-Phenyl-1,2,3,6-tetrahydropyridyl-1-methyl)-6-trifluormethyl-1,2,3,4-tetrahydrocarbazol
3-(4-Phenyl-1,2,3,6-tetrahydropyridyl-1-methyl)-7-trifluormethyl-1,2,3,4-tetrahydrocarbazol
3-(4-Phenyl-1,2,3,6-tetrahydropyridyl-1-methyl)-6-cyan-1,2,3,4-tetrahydrocarbazol
3-(4-Phenyl-1,2,3,6-tetrahydropyridyl-1-methyl)-7-cyan-1,2,3,4-tetrahydrocarbazol
3-(4-Phenyl-1,2,3,6-tetrahydropyridyl-1-methyl)-8-cyan-1,2,3,4-tetrahydrocarbazol
3-(4-Phenyl-1,2,3,6-tetrahydropyridyl-1-methyl)-6,7-dimethoxy-1,2,3,4-tetrahydrocarbazol, F. 178-180 °
3-(4-Phenyl-1,2,3,6-tetrahydropyridyl-1-methyl)-6,7-methylendioxy-1,2,3,4-tetrahydrocarbazol

3-(4-o-Tolyl-1,2,3,6-tetrahydropyridyl-1-methyl)-1,2,3,4-tetrahydrocarbazol
3-(4-m-Tolyl-1,2,3,6-tetrahydropyridyl-1-methyl)-1,2,3,4-tetrahydrocarbazol
3-(4-p-Tolyl-1,2,3,6-tetrahydropyridyl-1-methyl)-1,2,3,4-tetrahydrocarbazol
3-(4-o-Methoxyphenyl-1,2,3,6-tetrahydropyridyl-1-methyl)-1,2,3,4-tetrahydrocarbazol
3-(4-m-Methoxyphenyl-1,2,3,6-tetrahydropyridyl-1-methyl)-1,2,3,4-tetrahydrocarbazol
3-(4-p-Methoxyphenyl-1,2,3,6-tetrahydropyridyl-1-methyl)-1,2,3,4-tetrahydrocarbazol
3-(4-o-Hydroxyphenyl-1,2,3,6-tetrahydropyridyl-1-methyl)-1,2,3,4-tetrahydrocarbazol

3-(4-m-Hydroxyphenyl-1,2,3,6-tetrahydropyridyl-1-methyl)-1,2,3,4-tetrahydrocarbazol
3-(4-p-Hydroxyphenyl-1,2,3,6-tetrahydropyridyl-1-methyl)-1,2,3,4-tetrahydrocarbazol
3-(4-o-Fluorphenyl-1,2,3,6-tetrahydropyridyl-1-methyl)-1,2,3,4-tetrahydrocarbazol
3-(4-m-Fluorphenyl-1,2,3,6-tetrahydropyridyl-1-methyl)-1,2,3,4-tetrahydrocarbazol
3-(4-p-Fluorphenyl-1,2,3,6-tetrahydropyridyl-1-methyl-1,2,3,4-tetrahydrocarbazol
3-(4-o-Chlorphenyl-1,2,3,6-tetrahydropyridyl-1-methyl)-1,2,3,4-tetrahydrocarbazol
3-(4-m-Chlorphenyl-1,2,3,6-tetrahydropyridyl-1-methyl)-1,2,3,4-tetrahydrocarbazol
3-(4-p-Chlorphenyl-1,2,3,6-tetrahydropyridyl-1-methyl)-1,2,3,4-tetrahydrocarbazol
3-(4-p-Bromphenyl-1,2,3,6-tetrahydropyridyl-1-methyl)-1,2,3,4-tetrahydrocarbazol
3-(4-m-Trifluormethylphenyl-1,2,3,6-tetrahydropyridyl-1-methyl)-1,2,3,4-tetrahydrocarbazol
3-(4-p-Cyanphenyl-1,2,3,6-tetrahydropyridyl-1-methyl)-1,2,3,4-tetrahydrocarbazol
3-[4-(3,4-Dimethoxyphenyl)-1,2,3,6-tetrahydropyridyl-1-methyl]-1,2,3,4-tetrahydrocarbazol
3-[4-(3,4-Methylendioxyphenyl)-1,2,3,6-tetrahydropyridyl-1-methyl]-1,2,3,4-tetrahydrocarbazol
3-[4-(4-Chlor-3-trifluormethylphenyl)-1,2,3,6-tetrahydropyridyl-1-methyl]-1,2,3,4-tetrahydrocarbazol

3-[2-(4-Phenyl-1,2,3,6-tetrahydropyridyl)-ethyl]-1,2,3,4-tetrahydrocarbazol, Hydrochlorid, F. 263-265 °
3-[2-(4-Phenyl-1,2,3,6-tetrahydropyridyl)-ethyl]-6-methyl-1,2,3,4-tetrahydrocarbazol
3-[2-(4-Phenyl-1,2,3,5-tetrahydropyridyl)-ethyl]-6-methoxy-1,2,3,4-tetrahydrocarbazol, Hydrochlorid,

F. 214-216 °

3-[2-(4-Phenyl-1,2,3,6-tetrahydropyridyl)-ethyl]-7-methoxy-1,2,3,4-tetrahydrocarbazol, Hydrochlorid F. 258-260 °

3-[2-(4-Phenyl-1,2,3,6-tetrahydropyridyl)-ethyl]-6 methylthio-1,2,3,4-tetrahydrocarbazol

3-[2-(4-Phenyl-1,2,3,6-tetrahydropyridyl)-ethyl]-7 methylthio-1,2,3,4-tetrahydrocarbazol

3-[2-(4-Phenyl-1,2,3,6-tetrahydropyridyl)-ethyl]-5-hydroxy-1,2,3,4-tetrahydrocarbazol

3-[2-(4-Phenyl-1,2,3,6-tetrahydropyridyl)-ethyl]-6-hydroxy-1,2,3,4-tetrahydrocarbazol, Hydrochlorid F. 290-292 °

3-[2-(4-Phenyl-1,2,3,6-tetrahydropyridyl)-ethyl]-7-hydroxy-1,2,3,4-tetrahydrocarbazol, F. 206 - 208°

3-[2-(4-Phenyl-1,2,3,6-tetrahydropyridyl)-ethyl]-8-hydroxy-1,2,3,4-tetrahydrocarbazol

3-[2-(4-Phenyl-1,2,3,6-tetrahydropyridyl)-ethyl]-6-fluor-1,2,3,4-tetrahydrocarbazol

3-[2-(4-Phenyl-1,2,3,6-tetrahydropyridyl)-ethyl]-7-fluor-1,2,3,4-tetrahydrocarbazol

3-[2-(4-Phenyl-1,2,3,6-tetrahydropyridyl)-ethyl]-6-chlor-1,2,3,4-tetrahydrocarbazol

3-[2-(4-Phenyl-1,2,3,6-tetrahydropyridyl)-ethyl]-7-chlor-1,2,3,4-tetrahydrocarbazol, F. 182-184 °

3-[2-(4-Phenyl-1,2,3,6-tetrahydropyridyl)-ethyl]-6-brom-1,2,3,4-tetrahydrocarbazol

3-[2-(4-Phenyl-1,2,3,6-tetrahydropyridyl)-ethyl]-7-brom-1,2,3,4-tetrahydrocarbazol

3-[2-(4-Phenyl-1,2,3,6-tetrahydropyridyl)-ethyl]-6-trifluormethyl-1,2,3,4-tetrahydrocarbazol

3-[2-(4-Phenyl-1,2,3,6-tetrahydropyridyl)-ethyl]-7-trifluormethyl-1,2,3,4-tetrahydrocarbazol

3-[2-(4-Phenyl-1,2,3,6-tetrahydropyridyl)-ethyl]-6-cyan-1,2,3,4-tetrahydrocarbazol

3-[2-(4-Phenyl-1,2,3,6-tetrahydropyridyl)-ethyl]-7-cyan-1,2,3,4-tetrahydrocarbazol

3-[2-(4-Phenyl-1,2,3,6-tetrahydropyridyl)-ethyl]-8-cyan-1,2,3,4-tetrahydrocarbazol

3-[2-(4-Phenyl-1,2,3,6-tetrahydropyridyl)-ethyl]-6,7-dimethoxy-1,2,3,4-tetrahydrocarbazol, Hydrochlorid, F. 172-174 °

3-[2-(4-Phenyl-1,2,3,6-tetrahydropyridyl)-ethyl]-6,7-methylendioxy-1,2,3,4-tetrahydrocarbazol.

Beispiel 2

Ein Gemisch von 4,54 g 3-p-Toluolsulfonyloxymethyl-1,2,3,4-tetrahydrocarbazol und 3,18 g 4-Phenyl-1,2,3,6-tetrahydropyridin wird auf 130 ° erhitzt. Nach Abklingen der exothermen Reaktion und Erkalten arbeitet man wie üblich auf und erhält "P", F. 163-165 °.

Analog erhält man aus den entsprechenden Tosylaten:

3-(4-Phenyl-1,2,3,6-tetrahydropyridyl-1-methyl)-1-methyl-1,2,3,4-tetrahydrocarbazol

3-(4-Phenyl-1,2,3,6-tetrahydropyridyl-1-methyl)-2-methyl-1,2,3,4-tetrahydrocarbazol

3-(4-Phenyl-1,2,3,6-tetrahydropyridyl-1-methyl)-3-methyl-1,2,3,4-tetrahydrocarbazol

3-(4-Phenyl-1,2,3,6-tetrahydropyridyl-1-methyl)-4-methyl-1,2,3,4-tetrahydrocarbazol

3-(4-Phenyl-1,2,3,6-tetrahydropyridyl-1-methyl)-6-butyl-1,2,3,4-tetrahydrocarbazol

3-(4-Phenyl-1,2,3,6-tetrahydropyridyl-1-methyl)-6-butoxy-1,2,3,4-tetrahydrocarbazol

3-(4-Phenyl-1,2,3,6-tetrahydropyridyl-1-methyl)-6-butylthio-1,2,3,4-tetrahydrocarbazol.

Beispiel 3

Man kocht 3,1 g 3-Jodmethyl-1,2,3,4-tetrahydrocarbazol 1,59 g 4-Phenyl-1,2,3,6-tetrahydropyridin und 1,5 g wasserfreies Kaliumcarbonat in 25 ml n-Butanol 2 Stunden unter Rühren, läßt erkalten, arbeitet wie üblich auf und erhält "P", F. 163-165 °.

Analog erhält man mit den entsprechenden 4-Ar-1,2,3,6-tetrahydropyridinen:

3-(4-p-Butoxyphenyl-1,2,3,6-tetrahydropyridyl-1-methyl)-1,2,3,4-tetrahydrocarbazol
3-(4-p-Methylthiophenyl-1,2,3,6-tetrahydropyridyl-1-methyl)-1,2,3,4-tetrahydrocarbazol
3-(4-p-Butylthiophenyl-1,2,3,6-tetrahydropyridyl-1-methyl)-1,2,3,4-tetrahydrocarbazol
3-(4-p-Methylsulfinylphenyl-1,2,3,6-tetrahydropyridyl-1-methyl)-1,2,3,4-tetrahydrocarbazol
3-(4-p-Methylsulfonylphenyl-1,2,3,6-tetrahydropyridyl-1-methyl)-1,2,3,4-tetrahydrocarbazol.

Beispiel 4

Ein Gemisch von 2,0 g 3-Aminomethyl-1,2,3,4-tetrahydrocarbazol (erhältlich durch Reaktion von 3-Brommethyl-1,2,3,4-tetrahydrocarbazol mit Phthalimidkalium und anschließende Hydrolyse) und 2,15 g 1,5-Dichlor-3-phenyl-2-penten (erhältlich durch Reduktion von 3-Phenyl-2-penten-1,5-disäurediethylester mit $LiAlH_4$ und anschließende Reaktion mit $SOCl_2$) in 40 ml Aceton und 40 ml Wasser wird 24 Stunden gekocht und wie üblich

aufgearbeitet. Man erhält "P", F. 163-165 °.

Analog erhält man aus den entsprechenden Aminen und den entsprechenden 1,5-Dichlor-3-Ar-2-pentenen die anderen in den Beispielen 1, 2 und 3 angegebenen Verbindungen der Formel I.

Beispiel 5

Zu einer Lösung von 4,19 g 1-(1,2,3,4-Tetrahydrocarbazolyl-3-methyl)-4-phenyl-pyridiniumbromid (erhältlich aus 3-Brommethyl-1,2,3,4-tetrahydrocarbazol und 4-Phenyl-pyridin) in 50 ml 1n NaOH gibt man unter Rühren 1 g $NaBH_4$ in 20 ml Wasser und rührt danach noch 3 Stunden bei 60 °. Nach üblicher Aufarbeitung erhält man "P", F. 163-165 °.

Analog erhält man durch Reduktion der entsprechenden Pyridiniumbromide die anderen in den Beispielen 1, 2 und 3 angegebenen Verbindungen der Formel I.

Beispiel 6

Zu einer Suspension von 0,38 g $LiAlH_4$ in 10 ml THF tropft man eine Lösung von 3,56 g 3-(4-Phenyl-1,2,3,6-tetrahydropyridyl-carbonyl)-1,2,3,4-tetrahydrocarbazol (Rf 0,9; erhältlich aus 1,2,3,4-Tetrahydrocarbazol-3-carbonsäure und 4-Phenyl-1,2,3,6-tetrahydropyridin in Gegenwart von Carbonyldiimidazol in THF bei 20 °) in 10 ml THF unter Rühren. Nach Abklingen der Reaktion gibt man 5 ml Ethylacetat hinzu, arbeitet wie üblich auf und erhält "P", F. 163 - 165 °.

Analog erhält man aus den entsprechenden Säureamiden, z.B.

3-(4-Phenyl-1,2,3,6-tetrahydropyridyl-carbonyl)-6-methoxy-1,2,3,4-tetrahydrocarbazol (F. 210 - 212 °)

3-(4-Phenyl-1,2,3,6-tetrahydropyridyl-carbonyl)-7-methoxy-1,2,3,4-tetrahydrocarbazol (Rf 0,2, $CH_2Cl_2$/Methanol 98:2)

3-(4-Phenyl-1,2,3,6-tetrahydropyridyl-carbonyl)-6-hydroxy-1,2,3,4-tetrahydrocarbazol (F. 210 - 212 °)

3-(4-Phenyl-1,2,3,6-tetrahydropyridyl-carbonyl)-6,7-dimethoxy-1,2,3,4-tetrahydrocarbazol (F. 143 - 145 °)

3-(4-Phenyl-1,2,3,6-tetrahydropyridyl-carbonyl-methyl)-1,2,3,4-tetrahydrocarbazol (Rf 0,8, Toluol/$CH_3OH$/Triethylamin 7 : 2 : 1)

3-(4-Phenyl-1,2,3,6-tetrahydropyridyl-carbonyl-methyl)-6-methoxy-1,2,3,4-tetrahydrocarbazol (Rf 0,85)

3-(4-Phenyl-1,2,3,6-tetrahydropyridyl-carbonyl-methyl)-7-methoxy-1,2,3,4-tetrahydrocarbazol (Rf 0,6)

3-(4-Phenyl-1,2,3,6-tetrahydropyridyl-carbonyl-methyl)-6-hydroxy-1,2,3,4-tetrahydrocarbazol (Rf 0,85)

3-(4-Phenyl-1,2,3,6-tetrahydropyridyl-carbonyl-methyl)-7-chlor-1,2,3,4-tetrahydrocarbazol (Rf 0,9)

3-(4-Phenyl-1,2,3,6-tetrahydropyridyl-carbonyl-methyl)-6,7-dimethoxy-1,2,3,4-tetrahydrocarbazol (Rf 0,85)

die anderen in Beispiel 1 bis 3 angegebenen Verbindungen der Formel I.

Beispiel 7

Man kocht 4,82 g 9-Benzolsulfonyl-3-(4-Phenyl-1,2,3,6-tetrahydropyridyl-methyl)-1,2,3,4-tetrahydrocarbazol (erhältlich aus 9-Benzolsulfonyl-3-chlormethyl-1,2,3,4-tetrahydrocarbazol und 4-Phenyl-1,2,3,6-tetrahydropyridin) mit 1 g KOH in 7 ml Wasser und 14 ml Ethanol 16 Stunden, konzentriert das Gemisch, arbeitet wie üblich auf und erhält "P", F. 163-165 °.

Beispiel 8

Man erhitzt 3,74 g 3-(4-Hydroxy-4-phenyl-1-piperidyl-methyl)-9-methyl-1,2,3,4-tetrahydrocarbazol (erhältlich durch Reaktion von 3-Brommethyl-9-methyl-1,2,3,4-tetrahydrocarbazol mit 4-Piperidon, anschließende Umsetzung mit $C_6H_5Li$ und Hydrolyse) mit 40 ml 1n Salzsäure 2 Stunden auf 50 $^{o}$, arbeitet wie üblich auf und erhält 3-(4-Phenyl-1,2,3,6-tetrahydropyridyl-methyl)-9-methyl-1,2,3,4-tetrahydrocarbazol.

Beispiel 9

Zu einer siedenden Lösung von 3,88 g 3-(4-Phenyl-1,2,3,6-tetrahydropyridyl-1-methyl)-7-methylthio-1,2,3,4-tetrahydrocarbazol in 50 ml Ethanol gibt man 6 ml 30 %iges $H_2O_2$ und kocht anschließend 3 Stunden. Nach Zugabe weiterer 4 ml des Oxydationsmittels kocht man noch 9 Stunden, kühlt ab, arbeitet wie üblich auf und erhält 3-(4-Phenyl-1,2,3,6-tetrahydropyridyl-1-methyl)-7-methylsulfinyl-1,2,3,4-tetrahydrocarbazol.

Beispiel 10

Zu einer Lösung von 3,88 g 3-(4-Phenyl-1,2,3,6-tetrahydropyridyl-1-methyl)-7-methylthio-1,2,3,4-tetrahydrocarbazol in 20 ml Essigsäure gibt man 9 ml 30 %iges $H_2O_2$ und kocht 90 Minuten. Nach der üblichen Aufarbeitung erhält man 3-(4-Phenyl-1,2,3,6-tetrahydropyridyl-1-methyl)-7-methylsulfonyl-1,2,3,4-tetrahydrocarbazol.

**Beispiel 11**

**Ein Gemisch aus 4,19 g 3-(4-Phenyl-1,2,3,6-tetrahydro-pyridyl-1-methyl)-6-methoxy-1,2,3,4-tetrahydrocarbazol-hydrochlorid und 3,5 g Pyridinhydrochlorid wird 3 Stunden bei 160 ° gerührt. Nach üblicher Aufarbeitung erhält man 3-(4-Phenyl-1,2,3,6-tetrahydropyridyl-1-methyl)-6-hydroxy-1,2,3,4-tetrahydrocarbazol, Rf 0,2 (Toluol/Triethylamin 9:1).**

Beispiel 12

Analog Beispiel 6 erhält man aus den entsprechenden Säureamiden:

3-/4-(2-Thienyl)-1,2,3,6-tetrahydropyridyl-1-methyl7-1,2,3,4-tetrahydrocarbazol
3-(4-Phenyl-piperidinomethyl)-1,2,3,4-tetrahydrocarbazol
3-(4-Phenyl-piperidinomethyl)-6-hydroxy-1,2,3,4-tetrahydrocarbazol
3-(4-Phenyl-piperidinomethyl)-7-hydroxy-1,2,3,4-tetrahydrocarbazol
3-(4-m-Methoxyphenyl-piperidinomethyl)-1,2,3,4-tetrahydrocarbazol
3-(4-m-Hydroxyphenyl-piperidinomethyl)-1,2,3,4-tetrahydrocarbazol
3-(3-Phenyl-1,2,5,6-tetrahydropyridyl-1-methyl)-1,2,3,4-tetrahydrocarbazol
3-(3-Phenyl-1,2,5,6-tetrahydropyridyl-1-methyl)-6-hydroxy-1,2,3,4-tetrahydrocarbazol
3-(3-Phenyl-1,2,5,6-tetrahydropyridyl-1-methyl)-7-hydroxy-1,2,3,4-tetrahydrocarbazol
3-(3-m-Methoxyphenyl-1,2,5,6-tetrahydropyridyl-1-methyl)-1,2,3,4-tetrahydrocarbazol
3-(3-m-Hydroxyphenyl-1,2,5,6-tetrahydropyridyl-1-methyl)-1,2,3,4-tetrahydrocarbazol

3-(3-Phenyl-piperidinomethyl)-1,2,3,4-tetrahydrocarbazol
3-(3-Phenyl-piperidinomethyl)-6-hydroxy-1,2,3,4-tetrahydrocarbazol
3-(3-Phenyl-piperidinomethyl)-7-hydroxy-1,2,3,4-tetrahydrocarbazol
3-(3-m-Methoxyphenyl-piperidinomethyl)-1,2,3,4-tetrahydrocarbazol, Rf 0,65 ($CH_2Cl_2$/Methanol 9:1)
3-(3-m-Methoxyphenyl-piperidinomethyl)-6-methoxy-1,2,3,4-tetrahydrocarbazol, Rf 0,7 ($CH_2Cl_2$/Methanol 9:1)
3-(3-m-Hydroxyphenyl-piperidinomethyl)-1,2,3,4-tetrahydrocarbazol, F. 128-130°
3-(3-m-Hydroxyphenyl-piperidinomethyl)-6-hydroxy-1,2,3,4-tetrahydrocarbazol, F. 123-125°.

Beispiel 13

Eine Lösung von 1g 3-(3-m-Hydroxyphenyl-1,2,5,6-tetrahydropyridyl-1-methyl)-1,2,3,4-tetrahydrocarbazol in 15 ml Methanol wird an 1 g 5%igem Pd-C bei 1 bar und 20° hydriert. Man filtriert, dampft ein und erhält 3-(3-m-Hydroxyphenyl-piperidinomethyl)-1,2,3,4-tetrahydrocarbazol, F. 128-130°.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg 3-(4-Phenyl-1,2,3,6-tetrahydropyridyl-1-methyl)-1,2,3,4-tetrahydrocarbazol, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C Kapseln

2 kg 3-(4-Phenyl-1,2,3,6-tetrahydropyridyl-1-methyl)-6-hydroxy-1,2,3,4-tetrahydrocarbazol werden in üblicher Weise in Hartgelatinekapseln gefüllt, so daß jede Kapsel 20 mg des Wirkstoffs enthält.

Beispiel D: Ampullen

Eine Lösung von 1 kg 3-(4-Phenyl-1,2,3,6-tetrahydropyridyl-1-methyl)-7-hydroxy-1,2,3,4-tetrahydrocarbazol-hydrochlorid in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

0115607

Merck Patent Gesellschaft
mit beschränkter Haftung
D a r m s t a d t

Patentansprüche:

1. Tetrahydrocarbazolderivate der allgemeinen Formel I

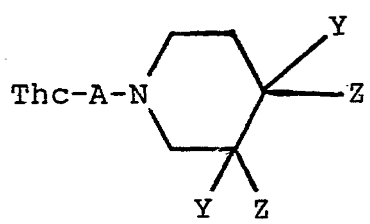

I

worin

Thc einen 1,2,3,4-Tetrahydrocarbazol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,

die beiden Reste Y jeweils H oder zusammen eine C-C-Bindung,

der eine Rest Z Ar,

der andere Rest Z H,

A $-CH_2-$ oder $-CH_2CH_2-$ und

Ar eine unsubstituierte oder ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituierte Phenylgruppe oder einen 2- oder 3-Thienylrest

bedeuten und

worin die Alkylgruppen jeweils 1 - 4 C-Atome besitzen,

sowie deren physiologisch unbedenkliche Säureadditionssalze.

2.a) 3-(4-Phenyl-1,2,3,6-tetrahydropyridyl-1-methyl)-1,2,3,4-tetrahydrocarbazol.

b) 3-(4-Phenyl-1,2,3,6-tetrahydropyridyl-1-methyl)-6-hydroxy-1,2,3,4-tetrahydrocarbazol.

c) 3-(4-Phenyl-1,2,3,6-tetrahydropyridyl-1-methyl)-7-hydroxy-1,2,3,4-tetrahydrocarbazol.

3. Verfahren zur Herstellung von Tetrahydrocarbazolderivaten der allgemeinen Formel I

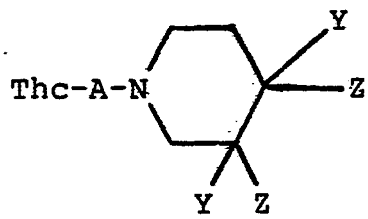

I

worin

Thc einen 1,2,3,4-Tetrahydrocarbazol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,

die beiden Reste Y jeweils H oder zusammen eine C-C-Bindung,

der eine Rest Z Ar,

der andere Rest Z H,

A $-CH_2-$ oder $-CH_2CH_2-$ und

Ar eine unsubstituierte oder ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituierte Phenylgruppe oder einen 2- oder 3-Thienylrest

bedeuten und

worin die Alkylgruppen jeweils 1 - 4 C-Atome besitzen,

sowie von dern physiologisch unbedenklichen Säureadditionssalzen, dadurch gekennzeichnet, daß man eine Verbindung der allgemeinen Formel II

$$Thc\text{-}A\text{-}X^1 \quad . \quad II$$

worin

$X^1$ X oder $NH_2$ und

X Cl, Br, J, OH oder eine reaktionsfähig funktionell abgewandelte OH-Gruppe bedeuten und

Thc und A die angegebenen Bedeutungen haben, mit einer Verbindung der allgemeinen Formel III

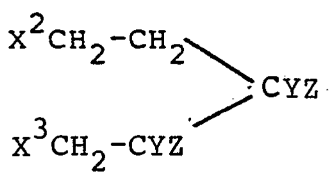

III

worin

$X^2$ und $X^3$ gleich oder verschieden sein können und, falls $X^1 = NH_2$ ist, jeweils X, andernfalls zusammen NH bedeuten und

Y und Z die angegebene Bedeutung haben

umsetzt

oder daß man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt

oder daß man eine sonst der Formel I entsprechende Verbindung, die jedoch an Stelle eines oder mehrerer Wasserstoffatome eine oder mehrere solvolytisch abspaltbare Gruppe(n) enthält,

mit einem solvolysierenden Mittel behandelt

oder daß man zur Herstellung von Verbindungen der Formel I, worin die beiden Reste Y zusammen eine C-C-Bindung bedeuten, eine Verbindung der Formel IV

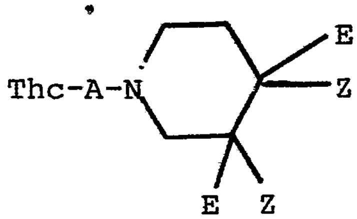

IV

worin

| | |
|---|---|
| der eine Rest E | X, CN oder $NH_2$, |
| der andere Rest E | H bedeutet und |
| Thc, A, X und Z | die angegebenen Bedeutungen haben |

mit einem HE-abspaltenden Mittel behandelt

und/oder daß man gegebenenfalls in einer Verbindung der Formel I eine Thioethergruppe zu einer SO-Gruppe oder $SO_2$-Gruppe oder eine SO-Gruppe zu einer $SO_2$-Gruppe oxydiert und/oder eine Alkoxygruppe unter Bildung einer OH-Gruppe spaltet und/oder eine C-C-Doppelbindung hydriert und/oder daß man eine erhaltene Base der Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Säureadditionssalze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Säureadditionssalze.

6. Verbindungen der allgemeinen Formel I nach Patentanspruch 1 zur Bekämpfung von Krankheiten.

7. Verwendung von Verbindungen der allgemeinen Formel I nach Patentanspruch 1 bei der Bekämpfung von Krankheiten.

8. Tetrahydrocarbazolderivate der allgemeinen Formel Va

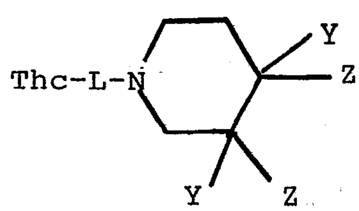

Va

worin

| | |
|---|---|
| L | -CO- oder $-CH_2CO-$ bedeutet und |
| Thc, Y und Z | die in Anspruch 1 angegebenen Bedeutungen haben. |

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0115607

Nummer der Anmeldung

EP 83 11 2690

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | US - A - 3 634 420 (R. LITTELL)<br>* Ganzes Dokument; Spalte 2, Zeilen 35,36 * | 1,4,5, 8 | C 07 D 401/06<br>C 07 D 409/14<br>C 07 D 491/04<br>A 61 K 31/445//<br>(C 07 D 491/04<br>317/00<br>209/00) |
| Y | US - A - 3 752 823 (J. MacMANUS)<br>* Ganzes Dokument; besonders Spalte 4, Formel IX, VII, Spalte 5, Formel XIII * | 1,4,5, 8 | |
| Y | GB - A - 1 299 041 (HOFFMANN LA ROCHE)<br>* Ganzes Dokument * | 1,4,5 | |
| | | | RECHERCHIERTE SACHGEBIETE (Int. Cl. 3) |
| PY | EP - A - 0 077 607 (BEECHAM)<br>* Ganzes Dokument * | 1,4,5 | C 07 D 401/00<br>C 07 D 409/00<br>C 07 D 491/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,8

Unvollständig recherchierte Patentansprüche:

Nicht recherchierte Patentansprüche: 6-7

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 30-03-1984 | NUYTS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82